# EUROPEAN PATENT APPLICATION

(11) **EP 3 766 469 A1**
(43) Date of publication of application: **20.01.2021**
(21) Application number: 20185286.0
(22) Date of filing: 10.07.2020
(51) Int. Cl.: A61G 7/018, A61B 5/00, A61G 7/05, A61G 7/057, G16H 40/63

(54) **PATIENT SUPPORT APPARATUS INCLUDING PATIENT RISK DETERMINATION SYSTEM**

(30) Priority: 16.07.2019 US 201962874589 P
(71) Applicant: Hill-Rom Services, Inc., Batesville, IN 47006-9167 (US)
(72) Inventor: YVERNAULT, Etienne, Batesville, Indiana 47006-9167 (US); THEPAUT, Anthony, Batesville, Indiana 47006-9167 (US); LE NAOUR, Pierre-Yves, Batesville, Indiana 47006-9167 (US); GAUTIER, Hervé, Batesville, Indiana 47006-9167 (US)
(74) Representative: Findlay, Alice Rosemary

(57) **Abstract**

A patient support apparatus includes a seat section, a head section rotatably coupled to the seat section, and a leg section rotatably coupled to the seat section to form a support surface for a patient. The support surface has a back area where the patient's back is positioned when the patient is positioned on the support surface, a leg area where the patient's legs are positioned when the patient is positioned on the support surface, and a foot area where the patient's legs are positioned when the patient is positioned on the support surface.

## Description

The present disclosure relates to patient support apparatuses. More particularly, the present disclosure relates to a hybrid air and foam mattresses.

Mattresses for patient support may include a plurality of air bladders. For example, the air bladders may include percussion or vibration therapy air bladders that assist in maintaining a comfort of the patient. The air bladders may also include turn-assist air bladders that assist in turning the patient. Each of the air bladders may be utilized to prevent pressure ulcers in the patient. A mobility of the patient is typically monitored to determine whether the patient is at risk for developing pressure ulcers.

When inflation of the air bladders is required, the air bladders are typically inflated with an air pump. The air pump may be formed integrally with the mattress. In other embodiments, the air pump may be formed integrally with a frame upon which the mattress rests. In yet other embodiments, the air pump may be a separate unit that is fluidly coupled to the mattress. The air bladders may be inflated with a single pump having valves to control air flow. Alternatively, different air bladders may be inflated with different air pumps.

The present disclosure includes one or more of the following features alone or in any combination.

According to an aspect of the disclosed embodiments, a patient support apparatus may include a seat section, a head section rotatably coupled to the seat section, and a leg section rotatably coupled to the seat section. The seat section, head section, and leg section may combine to form a support surface for a patient. The support surface may have a back area where the patient's back is positioned when the patient is positioned on the support surface, a leg area where the patient's legs are positioned when the patient is positioned on the support surface, and a foot area where the patient's legs are positioned when the patient is positioned on the support surface. At least one sensor may be positioned in at least one of the back area, leg area, and foot area. The at least sensor may determine at least one angle of a patient position on the support surface. A control system may be coupled to the at least one sensor to determine risk areas for the patient based on the at least one angle of the patient position. The risk areas may be areas of the patient that are prone to pressure sores.

In some embodiments, the at least one sensor may be positioned at the back area. The at least one angle of the patient position may be an angle of the patient's back relative to a floor. The at least one sensor may be positioned at the leg area. The at least one angle of the patient position may be an angle of the patient's legs relative to a floor. The at least one sensor may be positioned at the foot area. The at least one angle of the patient position may be an angle of the patient's feet relative to a floor. The at least one sensor may include a first sensor positioned in at least one of the back area, the leg area, and the foot area. A second sensor may be positioned in another of the back area, the leg area, or the foot area. The at least one sensor may include a first sensor positioned in the back area. A second sensor may be positioned in the leg area. A third sensor may be positioned in the foot area. The at least one sensor may include an accelerometer.

Optionally, at least one pressure sensor may be positioned in at least one of the back area, the leg area, and the foot area. The at least one pressure sensor may determine a pressure exerted on the patient. The control system may determine risk areas for the patient based on a determined pressure. The at least one pressure sensor may be positioned at the back area. The pressure exerted on the patient may be a pressure exerted on the patient's back. The at least one pressure sensor may be positioned at the leg area. The pressure exerted on the patient may be a pressure exerted on the patient's legs. The at least one pressure sensor may be positioned at the foot area. The pressure exerted on the patient may be a pressure exerted on the patient's feet. The at least one pressure sensor may include a first pressure sensor positioned in at least one of the back area, the leg area, and the foot area. A second pressure sensor may be positioned in another of the back area, the leg area, or the foot area. The at least one pressure sensor may include a first pressure sensor positioned in the back area. A second pressure sensor may be positioned in the leg area. A third pressure sensor may be positioned in the foot area.

According to another aspect of the disclosed embodiments, a patient support apparatus may include a mattress formed from a support material. A plurality of slots may be formed in the support material. A plurality of air bladders may be provided. Each air bladder may be positioned within a slot. A cavity may be formed in the support material. A pump may be configured to be positioned within the cavity. The pump may be fluidly coupled to the plurality of air bladders when the pump is positioned within the cavity. The pump may be configured to inflate and deflate the plurality of air bladders.

It may be contemplated that at least one manifold may have an inlet to receive air from the pump. The manifold may have a plurality of outlets to discharge the air into the plurality of air bladders. The at least one manifold may include a first manifold fluidly coupled to a first plurality of air bladders. A second manifold may be fluidly coupled to a second plurality of air bladders. The first manifold and the second manifold may each be fluidly coupled to the pump. Each air bladder of the first plurality of air bladders may be positioned between adjacent air bladder of the second plurality of air bladders. The first plurality of air bladders may be positioned in a first section of the mattress. The second plurality of air bladders may be positioned in a second section of the mattress.

It may be desired that the support material includes foam. The plurality of slots may be formed in the support material so that each slot of the plurality of slots is separated by a section of support material. Each slot of the plurality of slots may extend between a left side and a right side of the mattress. Each slot may extend from an opening formed in a first side of the mattress to a manifold positioned in a second side of the mattress. Each air bladder of the plurality of air bladders may be removably positioned within a respective slot. Each air bladder of the plurality of air bladders may be configured to be inserted into the opening formed in the first side of the mattress and fluidly coupled to the manifold positioned in the second side of the mattress.

Optionally, the pump may be removably inserted into the cavity. The cavity may extend from a cavity opening formed in a first side of the mattress to a manifold positioned in a second side of the mattress. The pump may fluidly couple to the manifold when the pump is positioned within the cavity.

According to yet another aspect of the disclosed embodiments, a patient support apparatus may include a mattress having a first side and a second side. A first turn-assist air bladder may be positioned on the first side. A second turn-assist air bladder may be positioned on the second side. A first inlet may extend through the first side and may be in fluid communication with the first turn-assist air bladder. A second inlet may extend through the second side and may be fluid communication with the second turn-assist air bladder. A pump may be configured to selectively couple to one of the first inlet and the second inlet.

In some embodiments, the pump may be a hands-free pump and may be operable to pump air without the use of hands. The pump may be foot activated and may be configured to position on the floor. A hose may extend from the pump. The hose may be configured to be coupled to one of the first inlet and the second inlet. The pump may be configured to couple to the first inlet to inflate the first turn-assist air bladder. The pump may be configured to couple to the second inlet to inflate the second turn-assist air bladder. The first turn-assist air bladder and the second turn-assist air bladder may be positioned within the mattress. The first turn-assist air bladder and the second turn-assist air bladder may be positioned under the mattress.

Optionally, a timer may track a period of time since a patient was turned. The timer may positioned within the mattress and may include a display. The timer may be configured to be set so that a reminder is issued to turn the patient after a predetermined time period. An input may be provided to select the predetermined time period. The timer may display a first color during the predetermined time period. The timer may display a second color after the expiration of the predetermined time period. The timer may reset after the patient is turned.

According to a further aspect of the disclosed embodiments, a patient support apparatus may include a mattress positioned on a frame. A plurality of air bladders may be positioned in the mattress and may be configured to be inflated and deflated to adjust a position of a patient on the mattress. A plurality of sensors may be configured to measure data related to the patient. A control system may be configured to track movement of the patient based on operation of the plurality of bladders and the data related to the patient. A display may be configured to display the movement tracked by the control system in a graph. A caregiver may review the graph to determine treatment for the patient.

It may be contemplate that at least one of the plurality of air bladders may be a therapeutic air bladder configured to provide pulsations or vibrations to the patient. The control system may track therapy provided for the patient and the graph may include an indicator representing the therapy. At least one of the plurality of air bladders may be a turn-assist air bladder configured to aid turning the patient. The control system may track patient turn assists and the graph may include an indicator representing patient turn assists. The graph may include a time stamp that identifies the time of a last patient turn assist.

It may be desired that an input may enable a caregiver to identify a type of patient care that resulted in the patient turn assist. The type of patient care may include at least one of turning the patient, repositioning the patient, patient out of bed, skin assessment, hygiene care, and wound care. The graph may include a label identifying the type of patient care.

In some embodiments, the plurality of sensors may include a pressure sensor. The pressure sensor may detect patient movement on the mattress based on a pressure detected by the pressure sensor. The pressure sensor may detect when the patient is exiting the mattress. At least one of the plurality of air bladders may be inflated to assist the patient in exiting the mattress.

Optionally, the display may indicate a patient mobility level. The patient mobility level may include at least one of tonic, weak, and inert. The patient mobility level may include a color coded graph. The display may be coupled to the frame. The display may include a remote display.

According to yet a further aspect of the disclosed embodiments, a patient support apparatus may include a frame. A control system may be coupled to the frame and may have a database including troubleshooting data. An input may enable a user to select troubleshooting data. A display may be configured to display the troubleshooting data. The troubleshooting data may include data related to maintenance of the patient support apparatus. The troubleshooting data may include an augmented reality display. The troubleshooting data may include instructional videos. The input may be positioned on the display. The display may be coupled to the frame. The display may be positioned on a mobile device.

The invention will now be further described by way of example with reference to the accompanying drawings, in which:
Fig. 1 is a side perspective view of a patient support apparatus in accordance with an embodiment;
Fig. 2 is a schematic view of angles measured on a mattress when a patient is positioned on the mattress;
Fig. 3 is a diagram illustrating risk areas in the patient and slip risks of the patient based on the measured angles of the mattress;
Fig. 4 illustrates additional diagrams of risk areas in the patient and slip risks of the patient based on the measured angles of the mattress;
Fig. 5 is a side perspective view of a patient mattress having slots extending from a first side to a second side and configured to receive air bladders;
Fig. 6 is a top schematic view of a mattress having alternating air bladders and foam sections, wherein each air bladder is coupled to a manifold that is in fluid communication with a pump;
Fig. 7 is a top schematic view of a mattress having alternating air bladders and foam sections, wherein each air bladder is coupled to one of two manifolds that are in fluid communication with a pump;
Fig. 8 is a side perspective view of a patient support apparatus having a mattress with turn-assist bladders and a hands-free, foot-operated air pump for inflating the turn-assist bladders;
Fig. 9 is a flowchart for monitoring a time period between patient turns on a patient support apparatus;
Fig. 10 is a screen showing an exemplary display of monitored patient movement on a patient support apparatus;

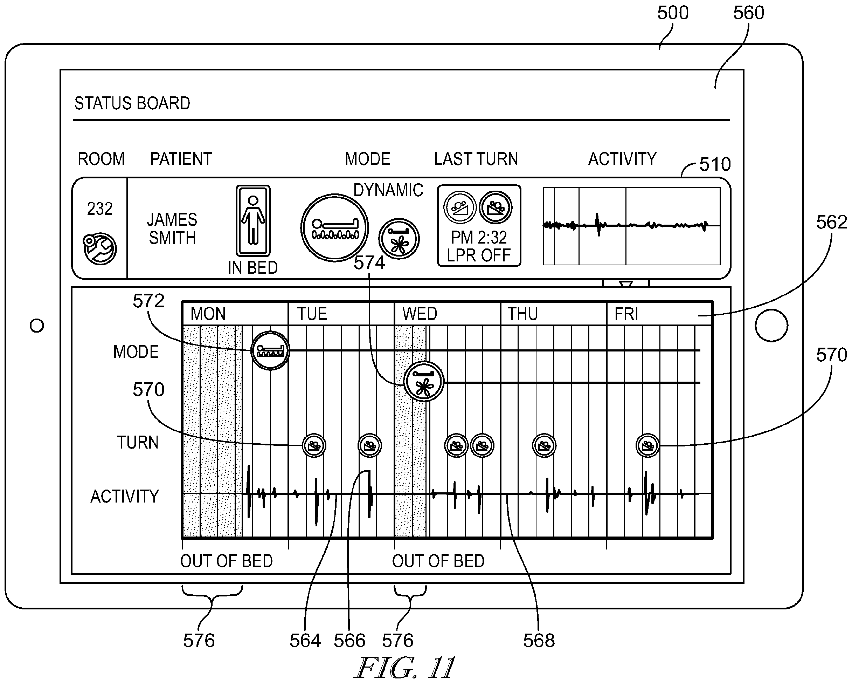

Fig. 11 is a screen showing another exemplary display of monitored patient movement on a patient support apparatus;
Fig. 12 is a flowchart of patient displays related to a patient's mobility;
Fig. 13 is another flowchart of patient displays related to a patient's mobility;
Fig. 14 is a screen showing an exemplary display for tracking patient care related to turn assists;
Fig. 15 is a side perspective view of a foot end of a patient support apparatus having a control system with a database for troubleshooting the patient support apparatus;
Fig. 16 is a screen on a mobile device displaying an augmented reality related to troubleshooting the patient support apparatus; and
Fig. 17 is a screen on the control system displaying an augmented reality related to troubleshooting the patient support apparatus.

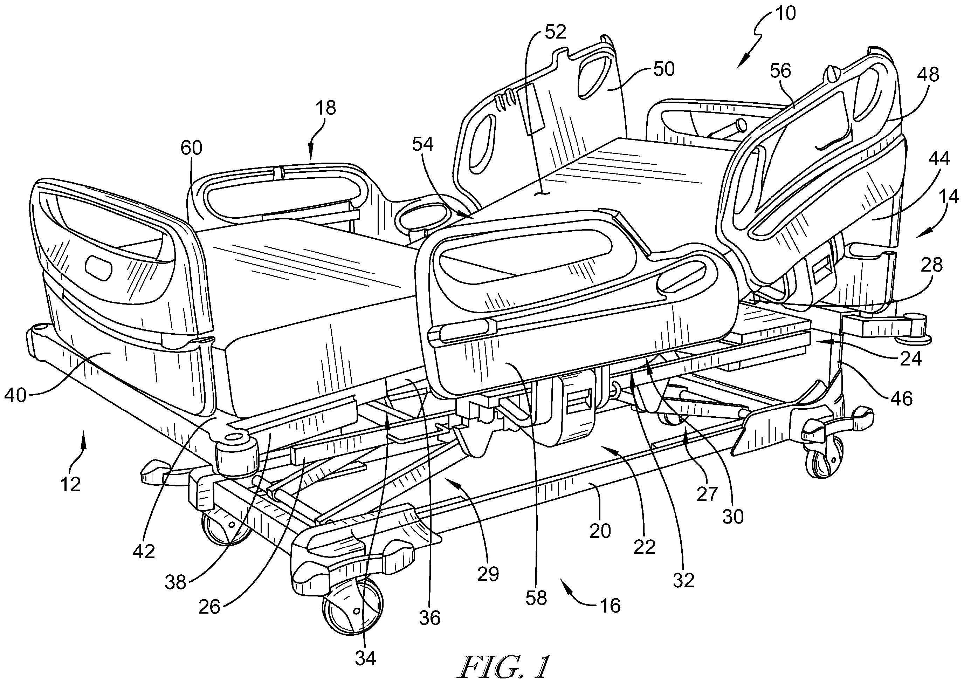

Referring to Fig. 1, a patient support apparatus 10 is illustratively embodied as a hospital bed 10. The view shown in Fig. 1 is generally taken from a position that is oriented at the left side, foot end of the hospital bed 10. For purposes of orientation, the discussion of the hospital bed 10 will be based on the orientation of a patient supported on the hospital bed 10 in a supine position. Thus, the foot end 12 of the hospital bed 10 refers to the end nearest the patient's feet when the patient is supported on the hospital bed 10 in the supine position. The hospital bed 10 has a head end 14 opposite the foot end 12. A left side 16 refers to the patient's left when the patient is lying in the hospital bed 10 in a supine position. The right side 18 refers to the patient's right. When reference is made to the longitudinal length of the hospital bed 10, it refers a direction that is represented by the lines that generally extend between the head end 14 and foot end 12 of the hospital bed 10. Similarly, lateral width of the hospital bed 10 refers to a direction that is represented by the lines that generally extend between the left side 16 and right side 18.

The hospital bed 10 includes a base frame 20, which supports a lift system 22. The lift system 22 engages the base and an upper frame 24 such that the lift system 22 moves the upper frame 24 vertically relative to the base frame 20. The lift system 22 includes a head end linkage 27 and a foot end linkage 29. Each of the linkages 27 and 29 are independently operable and may be operated to cause the hospital bed 10 to move into a tilt position which is when the head end 14 of the upper frame 24 is positioned lower than the foot end 12 of the upper frame 24. The hospital bed 10 may also be moved to a reverse tilt position with the foot end 12 of the upper frame 24 is positioned lower than the head end 14 of the upper frame 24.

The upper frame 24 supports a load frame 26. The load frame 26 supports a head deck 28 which is movable relative to the load frame 26. The load frame 26 also supports an articulated seat deck 30, also movable relative to the load frame 26 and a fixed seat deck 32. Also supported from the load frame 26 is a foot deck 34 that is articulated and moveable relative to the load frame 26. The foot deck 34 in the illustrative embodiment of Fig. 1 provides for powered pivoting of the foot deck 34 and manual extension and retraction of the foot deck 34 to vary the length of the foot deck 34. In other embodiments, powered pivoting of the foot deck 34 may be omitted and the related movement may be caused manually, or follow movement of the articulated seat deck 30. In addition, in some embodiments, extension and retraction of the foot deck 34 may be powered by an actuator.

The foot deck 34 includes a first portion 36 and a second portion 38, which moves relative to the first portion 36 to vary the size of the foot deck 34. The second portion 38 moves generally longitudinally relative to the first portion 36 to vary the longitudinal length of the foot deck 34 and, thereby, the longitudinal length of the hospital bed 10.

A foot panel 40 is supported from the second portion 38 and extends vertically from an upper surface 42 of the second portion 38 to form a barrier at the foot end 12 of the hospital bed 10. A head panel 44 is positioned on an upright structure 46 of the base frame 20 and extends vertically to form a barrier at the head end 14 of the hospital bed 10. A left head side rail 48 is supported from the head deck 28 and is moveable between a raised position shown in Fig. 1 and a lowered position as is known in the art. A right head side rail 50 is also moveable between the raised position of Fig. 1 and lowered position. As shown in Fig. 1, in the raised position, the side rails 48 and 50 extend above an upper surface 52 of a mattress 54 of the hospital bed 10 when the side rails 48 and 50 are in a raised position. In a lowered position an upper edge 56 of the left head side rail 48 is below the upper surface 52. It should be appreciated that in some embodiments, the left head side rail 48 and the right head side rail 50 are movable to a position between the raised position and the lowered position.

The hospital bed 10 also includes a left foot side rail 58 and a right foot side rail 60, each of which is supported directly from the load frame 26. Each of the side rails 48, 50, 58, and 60 are operable to be lowered to a position below the upper surface 52. It should be appreciated that in some embodiments, the left foot side rail 58 and the right foot side rail 60 are movable to a position between the raised position and the lowered position. It should be noted that when the head deck 28 is moved, the head side rails 48 and 50 move with the head deck 28 so that they maintain their relative position to the patient. This is because both of the head side rails 48 and 50 are supported by the head deck 28.

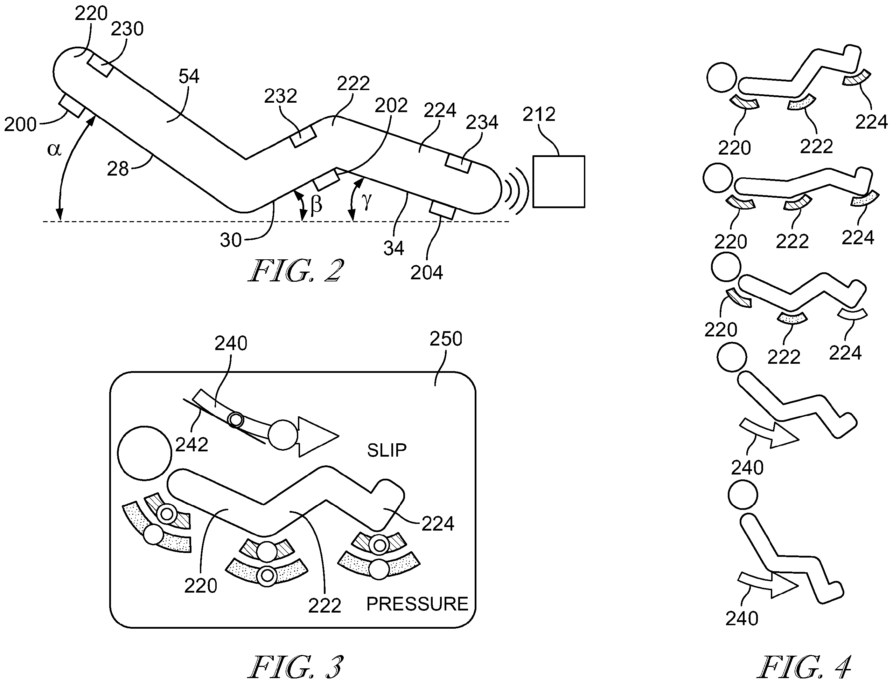

Fig. 2 illustrates the mattress 54 in a non-flat configuration. The head deck 28 is rotated relative to the seat deck 30, and foot deck 34 is rotated relative to the seat deck 30. A sensor 200 is positioned in the head deck 28. The sensor 200 may be an accelerometer configured to measure an angle α of the head deck 28 relative to a floor 210. A sensor 202 is positioned in the seat deck 30. The sensor 202 may be an accelerometer configured to measure an angle β of the seat deck 30 relative to the floor 210. A sensor 204 is positioned in the foot deck 34. The sensor 204 may be an accelerometer configured to measure an angle γ of the foot deck 34 relative to the floor 210.

The sensors 200, 202, 204 send signals to a control system 212 that includes a processor 214 configured to determine risk areas for the patient based on data in the signals. The mattress 54 includes a back area 220 where a patient's back is positioned when resting on the mattress 54. A leg area 222 provides a place for the patient's legs to rest when resting on the mattress 54. The patient's feet rest in a foot area 224 of the mattress 54. The control system 212 determines risk areas in the back area 220, the leg area 222, and the foot area 224 based on the signals from the sensors 200, 202, 204. A risk area may occur when one of the back area 220, the leg area 222, or the foot area 224 is becomes prone to ulcers or pressure sores. In some embodiments, the mattress 54 also includes pressure sensors. For example, the pressure sensors may include a pressure sensor 230 in the back area 220, a pressure sensor 232 in the seat area 222, and/or a pressure sensor 234 in the foot area 224.

The control system 212 utilizes data from the pressure sensors 230, 232, and 234 to determine risk areas for the patient. In some embodiments, the risk areas may be determined by only the pressures sensors 230, 232, and 234. In some embodiments, the risk area may be determined by only the sensors 200, 202, 204. In some embodiments, the risk areas are determined by control system 212 using data from both the sensors 200, 202, 204 and the pressures sensors 230, 232, and 234. A display may illustrate the mattress 54 and the risk areas using colors. For example, green may indicate that the patient is at low risk in the current position. Yellow may indicate that the patient is at some risk in the current position, and red may indicate that the patient is at high risk in the current position. Risk levels may be illustrated at each of the back area 220, the seat area 222, and/or the foot area 224. For example, as shown on the display 250 in Fig. 3, the back area 220 may be illustrated in green, indicating low risk; the seat area 222 may be indicated in red, indicating high risk; and the foot area 224 may be indicated in yellow, indicating moderate risk. The display may also illustrate an arrow 240 having slope 242. The slope 242 of the arrow 240 is indicative of the patient's probability of sliding on the mattress 54 toward a foot end of the mattress 54. Fig. 4 shows various exemplary color coded illustrations that may be shown on a display 250. It should be noted that the display 250 may be provided on the apparatus 10 and/or on a remote device.

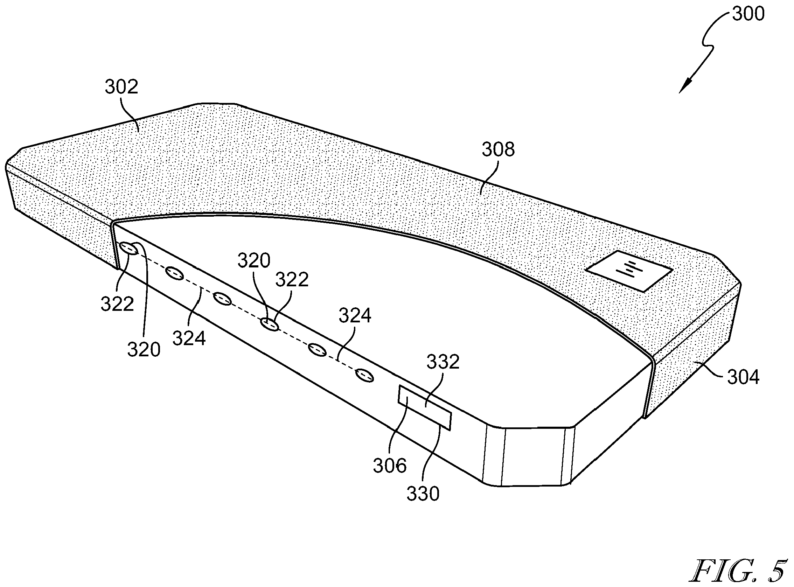

Referring now to Fig. 5, a mattress 300 is usable with the bed 10. The mattress 300 is formed from a supportive material such as foam. The mattress 300 includes a head end 302 and a foot end 304. A first side 306 extends between the head end 302 and the foot end 304. Additionally, a second side 308 extends between the head end 302 and the foot end 304 opposite the first side 306.

A plurality of slots 320 extend from openings 322 in the first side 306. Notably, the slots 320 could be formed in the second side 308. The slots 320 extend from the openings 322 into the mattress 300. A section of supportive material 324 is positioned between each slot 320. That is, slots 320 and the sections of supportive material 324 alternate along the first side 306. A cavity 330 also extends from an opening 332 in the first side 306. The cavity 330 extends from the opening 332 into the mattress 300. It should be appreciated that the cavity 330 may extend from an opening 332 in the second side 308.

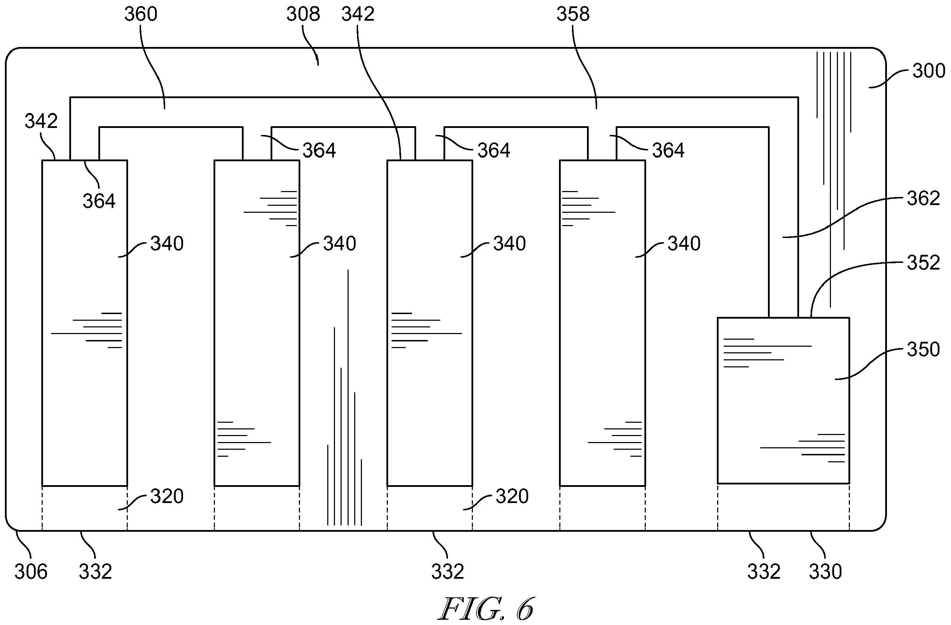

As illustrated in Fig. 6, a plurality of air bladders 340 are positioned in the plurality of slots 320. Each air bladder 340 of the plurality of air bladders is positioned in one of the slots 320 of the plurality of slots. The air bladders 340 are configured to be removably inserted into the slots 320. The air bladders 340 extend from the opening 332 of the slot 320 to an end 342 of the slot 320. A pump 350 is configured to be removably inserted into the cavity 330. The pump 350 is positioned between the opening 332 and an end 352 of the cavity 330. The pump 350 may be inserted into the cavity 330, when the patient requires the air bladders 340. When the air bladders 340 are not in use, the pump 350 may be removed from the cavity 330. Alternatively, the pump 350 may be positioned in the cavity 330 at any time that the mattress 300 is in use.

A manifold 360 is positioned in the second side 308 of the mattress 300. The manifold 360 includes tubing 358 that extends from an inlet 362 to a plurality of outlets 364. The inlet 362 is positioned in the end 352 of the cavity 330. The pump 350 is configured to fluidly couple to the inlet 362 when the pump 350 is inserted into the cavity 330. Each of the outlets 364 is positioned in an end 342 of each slot 320. The air bladders 340 are configured to fluidly couple to an outlet 364 when the air bladders 340 are positioned in the respective slots 320. When the pump 350 and the air bladders 340 are positioned in the mattress 300, the air bladders 340 may be inflated and deflated by the pump 350. The air bladders 340 may be inflated to firm the mattress 300. Also, the air bladders 340 may be inflated and deflated to provide percussion or vibration therapy to the patient. The pump 350 may be powered by a power cord extending from the cavity 330 and configured to plug into an outlet. Alternatively, the pump 350 may be powered by a power supply provided within the mattress 300. The pump 350 may be electrically coupled to the mattress 300, when the pump 350 is inserted into the cavity 330.

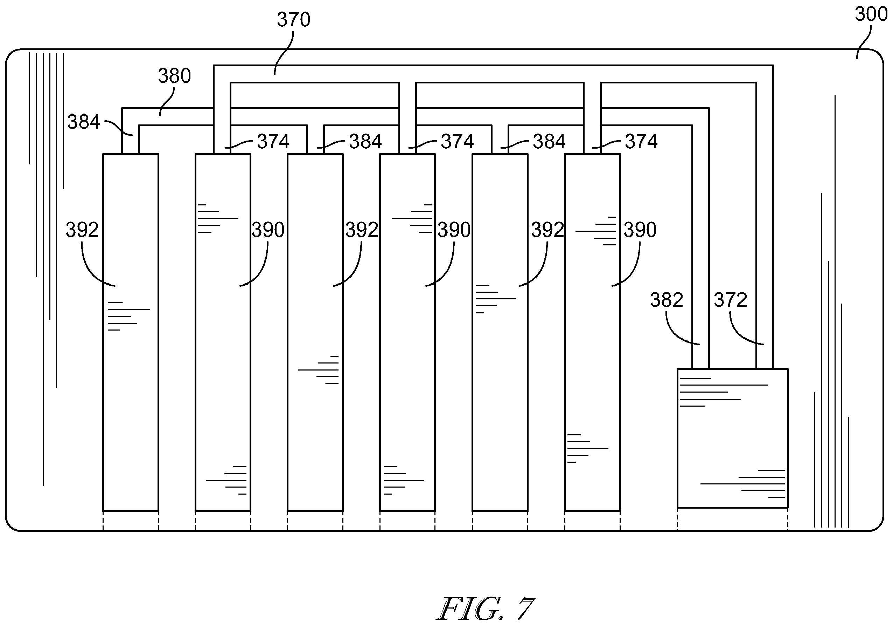

Referring to Fig. 7, an embodiment of the mattress 300 includes a first manifold 370 having an inlet 372 and a plurality of outlets 374, and a second manifold 380 having an inlet 382 and a plurality of outlets 384. Each of the inlets 372 and 382 are configured to fluidly couple to the pump 350. The outlets 374 are each coupled to one of a first plurality of air bladders 390. The outlets 384 are each coupled to one of a second plurality of air bladders 392. The air bladders 390 and 392 are configured to be independently inflated and deflated by the pump 350. In the embodiment shown in Fig. 7, each of the first plurality of air bladders 390 alternates with each of the second plurality of air bladders 392. Accordingly, the bladders 390 and the bladders 392 may be alternately inflated and deflated to provide percussion or vibration therapy to the patient. In other embodiments, the first plurality of air bladders 390 are positioned in a first section of the mattress 300, and the second plurality of air bladders 392 are positioned in a second section of the mattress 300. For example, one plurality of air bladders may be positioned in one of a head section, seat section, or foot section of the mattress, and another plurality of air bladders may be positioned in another of the head section, seat section, or foot section.

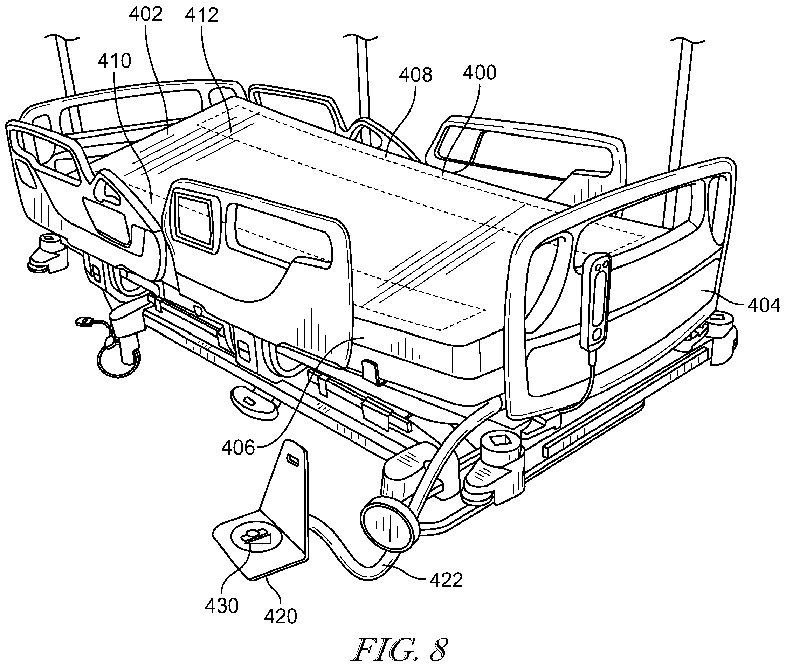

Figure 8 illustrates another embodiment of a mattress 400 having a head end 402 and a foot end 404. A first side 406 extends between the head end 402 and the foot end 404. A second side 408 extends between the head end 402 and the foot end 404 opposite the first side 406. The first side 406 includes a turn-assist bladder 410, and the second side 408 includes a turn-assist bladder 412. The turn assist bladders 410 and 412 are configured to inflate the first side 406 and the second side 408 of the mattress 400 to assist a caregiver in turning the patient.

A pump 420 is provided to inflate the turn-assist bladder 410 and 412. The pump 420 is configured to be fluidly coupled to the mattress 400, when one of the turn-assist bladders 410 or 412 requires inflation. For example, if the turn-assist bladder 410 requires inflation, a hose 422 extending from the pump 420 is inserted into the first side 406 of the mattress 400 and fluidly coupled to the turn-assist bladder 410. If the turn-assist bladder 412 requires inflation, the hose 422 is inserted into the second side 408 of the mattress 400 and fluidly coupled to the turn-assist bladder 412.

The pump 420 is configured to only be fluidly coupled to the mattress 400 when turn-assist is required. That is, the pump 420 may be stored at various locations within a healthcare facility, and attached to the mattress 400 when the patient needs to be turned. The pump 420 may be powered from an outlet in the healthcare facility or the pump 420 may be powered by the mattress 400 and/or the bed 10. The pump 420 is a hands-free pump that is configured to be operated by a caregiver's foot. The pump 420 rests on the floor and includes a switch 430 that may be operated by the caregiver's foot. Accordingly, the caregiver may use both hands to move the patient, while inflating the turn-assist bladder 410 or 412 with the caregiver's foot.

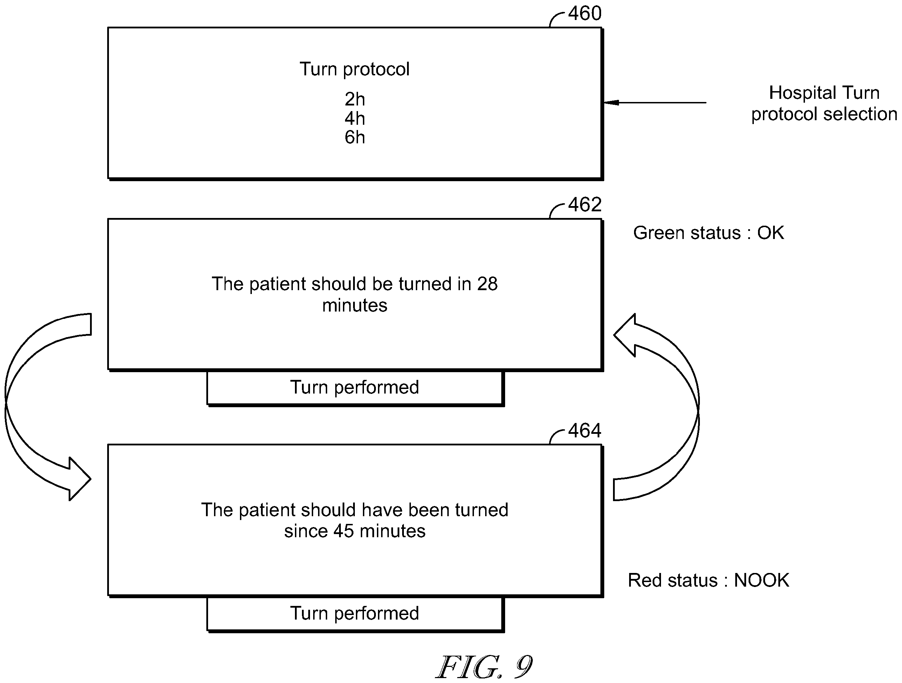

The bed 10 may include a display that tracks when the patient has been turned. Optionally, the display may be provided on a remote device, for example, a remote computer, phone, or tablet. Patient turning may be tracked using the flowchart illustrated in Fig. 9. At a first block 460, the caregiver may select a protocol for turning the patient. For example, the patient may be turned every 2 hours, 4 hours, or 6 hours. Other time periods may be contemplated. The display may indicate a time period until the patient requires turning, at block 462. For example, the display may indicate that the patient should be turned in 28 minutes. So long as the time period for turning the patient has not expired, the display may illustrate the remaining time in a first color, e.g. green. If the patient is not turned within the selected time period, the display indicates, at block 464 how much time has passed since the patient should have been tumed. For example, the display may indicate that the patient should have been turned 45 minutes ago. The display may indicate this warning in a second color, e.g. red.

Each time that the patient is turned, the timer is reset. For example, after turning the patient, a caregiver may manually reset the timer. Optionally, the timer may automatically reset when one of the turn-assist bladders 410 or 412 is inflated. It should be noted that the time period for turning the patient may be altered at any time. For example, if the patient begins to develop pressure sores, the caregiver may choose to select a shorter time period for turning the patient. Also, if the patient is sleeping, the caregiver may select a longer time period for turning the patient.

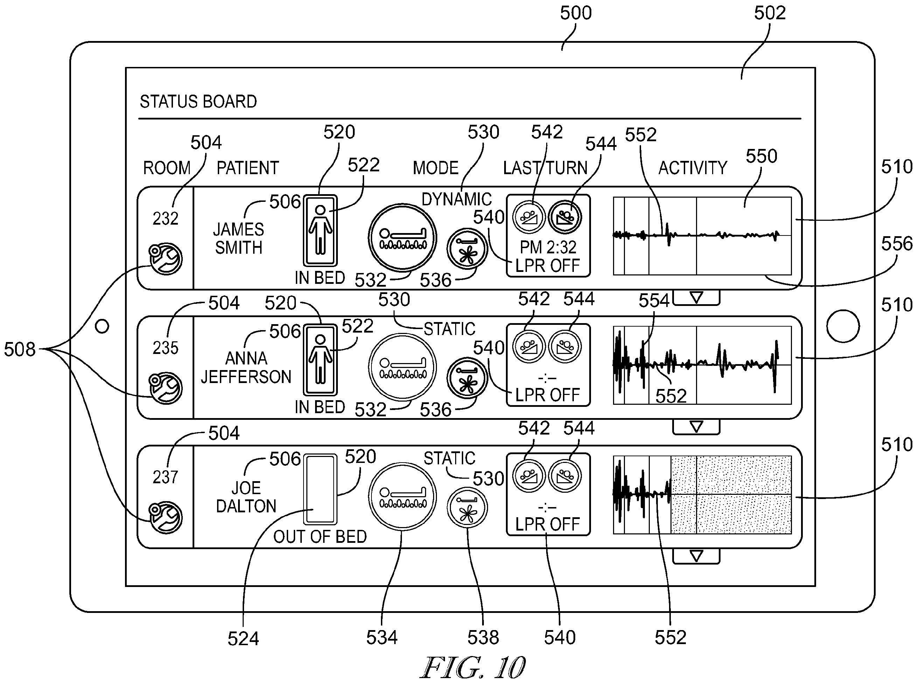

Fig. 10 illustrates a display 500 that may be utilized to track a patient's movement on the bed 10. The display 500 includes a status screen 502 that lists patients in a healthcare facility in individual patient status bars 510. For example, the screen 502 may list all of the patients in the healthcare facility. In some embodiments, the screen 502 may list only the patients for whom a nurse or caregiver is responsible. An indicator 504 lists the room that a patient is in next to a patient name block 506 that provides the patient's name. A settings icon 508 may be selected to change information related to the patient.

The screen 502 provides a summary of each patients movement. An icon 520 indicates whether the patient is in bed. For example, the icon 522 is illuminated, indicating that the patient is in bed. Conversely, the icon 524 is not illuminated, which indicates that the patient is not in bed. Mode icons 530 indicate a mode, in which the bed 10 is currently operating. The mode icon 530 may read "dynamic" or "static." "Static" indicates that the patient is not utilizing any of the bladder features of the bed 10. "Dynamic" indicates that the patient is using bladder features, such as percussion or vibration. The illuminated icon 532 indicates that a dynamic feature is in use. Conversely, the non-illuminated icon 534 indicates that a dynamic feature is not in use. Additionally, an illuminated icon 536 indicates that a micro-climate management system of the bed 10 is in use to cool the patient. A non-illuminated icon 538 indicates that the micro-climate management system is not in use.

A tum-assist indicator 540 illustrates when a patient has last been turned. For example, the indicator 540 includes a right-turn indicator 542 and a left-turn indicator 544. When the right-turn indicator 542 is illuminated, the patient has last been turned on their right side at the time shown in the turn-assist indicator 540. When the left-turn indicator 544 is illuminated, the patient has last been turned on their left side at the time shown in the turn-assist indicator 540. Neither indicator 542 or 544 being illuminated is indicative of the patient having not been turned.

A chart 550 tracks the patient's movement over time 556. The chart 550 includes an activity line 552 that indicates a magnitude of patient movement at specific times. When the line 552 is flat, the line indicates that the patient was not moving for the time period that the line 552 is flat. Peaks 554 in the line 552 indicate movement at a particular time. A magnitude of each peak indicates a magnitude of the movement.

A caregiver make select a patient from the screen 502 to view a more detailed breakdown of a patient's movement in a detailed screen 560, shown in Fig, 11. The detailed screen 560 includes the individual patient status bar 510 and a detailed graph 562. The detailed graph 562 includes an activity line 564 that indicates the patient's movement over time 568. Generally, the activity line 564 is an enlarged view of the activity line 552. The activity line 564 includes flat sections 568 that indicate no movement and peaks 566 that indicate movement and a magnitude of the movement. The activity line 564 may be identical to the activity line 552. In other embodiment, due to being enlarged, the activity line 564 may include additional features not shown in the activity line 552, e.g. additional peaks.

The graph 562 also includes icons 570 that indicate the times that the patient was turned. Moreover, a line 572 indicates a time period in which the patient utilized the dynamic features of the bed 10. A line 574 indicates a time period that the patient used the micro-climate management system of the bed 10. The graph 562 also illustrates time periods that the patient is out of bed with shaded areas 576.

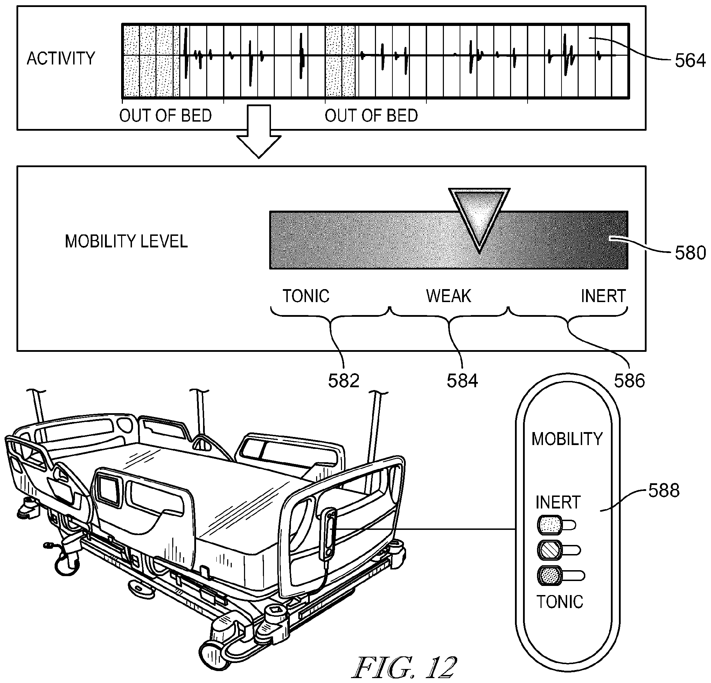

Referring to Fig. 12, the activity line 564 may be utilized to indicate a mobility level of the patient. That is, a control system monitors the activity line 564 and, based on an amount of patient movement and a magnitude of patient movement, assigns a mobility level to the patient. The mobility level is presented in a color-coded graph 580. Patients in a green area 582 of the graph 580 are considered to have a tonic mobility level. Patients in a yellow area 584 of the graph 580 are considered to have a weak mobility level. Patients in a red area 586 of the graph 580 are considered to have an inert mobility level. The graph 580 may be displayed on the bed 10 or on a remote display, e.g. a display of a caregiver's remote device. In some embodiments, a display 588 may only include a green, yellow, and red light to indicate the patient's mobility.

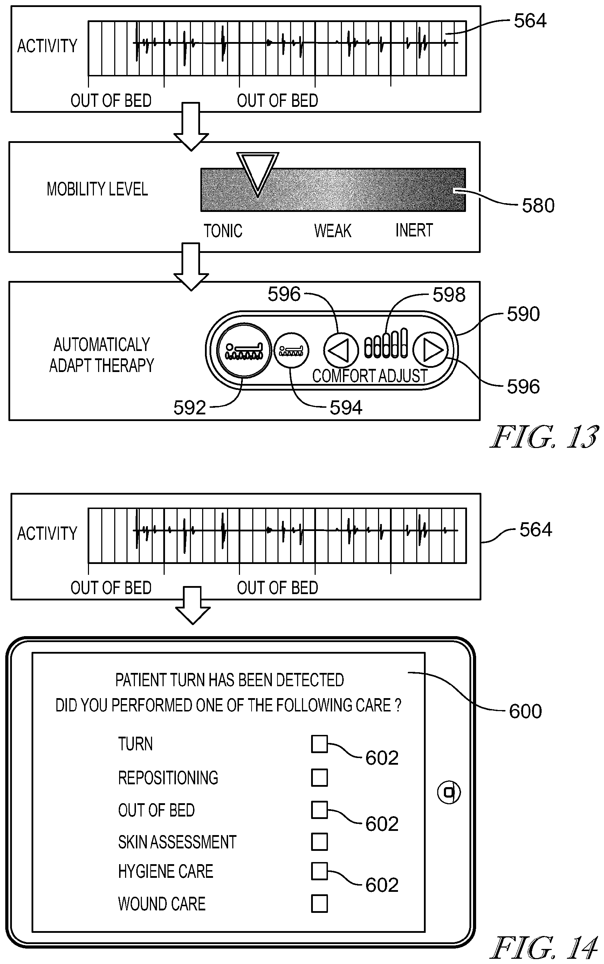

As illustrated in Fig. 13, a control 590 may be provided to adjust the patient's therapy based on the patient's mobility level. For example, the control 590 may include a button 592 for turning on patient therapy, e.g. percussion or vibration, and a button 594 for turning off patient therapy. Buttons 596 may be utilized to adjust an intensity of the therapy. The intensity may be displayed on a graph 598. If the activity line 564 indicates that the patient is likely getting out of the bed, air bladders within the bed may be inflated to assist the patient in exiting the bed. In some embodiments, the air bladders may be automatically inflated when bed exit is detected.

Referring to Fig. 14, a screen 600 may be provided for the caregiver to select a reason for patient care to keep complete records of the patient movement. The activity line 564 includes the icons 570 that indicate the times that the patient is turned. By selecting an icon 570, the screen 600 appears. The screen 600 provides check boxes 602 that may be selected to indicate a type of patient care provided. The types of patient care may include turning the patient, repositioning the patient, patient out of bed, skin assessment, hygiene care, and/or wound care. It should be noted that other types of patient care may be contemplated.

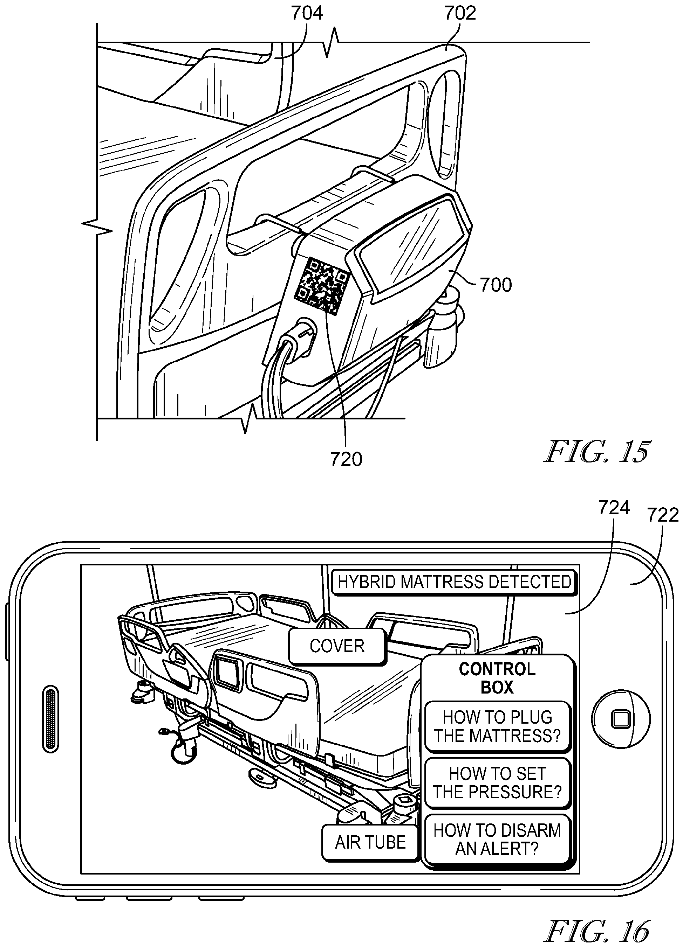

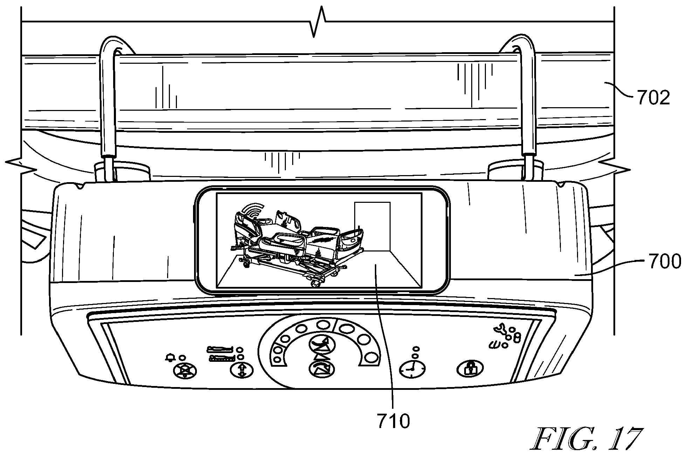

Referring now to Fig. 15, a control system 700 is coupled to a frame 702 of a bed 704, e.g. bed 10. The control system 700 may be removably coupled to the frame 702. The control system 700 includes a memory (not shown) and a processor (not shown) configured to carry out instructions in the memory to display videos and augmented reality related to the bed 704. The videos and augmented reality include maintenance instructions and troubleshooting guides related to the bed 704. The videos and augmented reality may be shown on a display 710 provided on the control system 700, as shown in Fig. 17.

Optionally, referring back to Fig. 15, the control system 700 includes readable code 720, e.g. a QR code, that is readable by a remote device 722, for example, a mobile phone or mobile device, as illustrated in Fig. 16. The readable code 720 is specific to a model of the bed 704. When the remote device 722 reads the code 720, the videos and augmented reality become available on a display 724 of the remote device 722 through a downloadable program that is stored on the remote device 722. In some embodiments, the remote device 722 may be a remote computer, e.g. a computer at a nurse's station, that is loaded with the videos and augmented reality. Accordingly, a caregiver can troubleshoot problems with the bed 704 via the videos and augmented reality.

Embodiments of the invention can be described with reference to the following numbered clauses, with preferred features laid out in the dependent clauses:
1. A patient support apparatus comprising:
   a seat section,
   a head section rotatably coupled to the seat section, and
   a leg section rotatably coupled to the seat section, wherein the seat section, head section, and leg section combine to form a support surface for a patient, the support surface having a back area where the patient's back is positioned when the patient is positioned on the support surface, a leg area where the patient's legs are positioned when the patient is positioned on the support surface, and a foot area where the patient's legs are positioned when the patient is positioned on the support surface,
   at least one sensor positioned in at least one of the back area, leg area, and foot area, the at least sensor determining at least one angle of a patient position on the support surface, and
   a control system coupled to the at least one sensor to determine risk areas for the patient based on the at least one angle of the patient position.
2. The patient support apparatus of clause 1, wherein the at least one sensor is positioned at the back area, and the at least one angle of the patient position is an angle of the patient's back relative to a floor.
3. The patient support apparatus of either clause 1 or clause 2, wherein the at least one sensor is positioned at the leg area, and the at least one angle of the patient position is an angle of the patient's legs relative to a floor.
4. The patient support apparatus of any preceding clause, wherein the at least one sensor is positioned at the foot area, and the at least one angle of the patient position is an angle of the patient's feet relative to a floor.
5. The patient support apparatus of any preceding clause, wherein the at least one sensor includes:
   a first sensor positioned in at least one of the back area, the leg area, and the foot area, and
   a second sensor positioned in another of the back area, the leg area, or the foot area.
6. The patient support apparatus of any preceding clause, wherein the at least one sensor includes:
   a first sensor positioned in the back area,
   a second sensor positioned in the leg area, and
   a third sensor positioned in the foot area.
7. The patient support apparatus of any preceding clause, wherein the at least one sensor includes an accelerometer.
8. The patient support apparatus of any preceding clause, further comprising at least one pressure sensor positioned in at least one of the back area, the leg area, and the foot area, the at least one pressure sensor determining a pressure exerted on the patient.
9. The patient support apparatus of clause 8, wherein the control system determines risk areas for the patient based on a determined pressure.
10. The patient support apparatus of either clause 8 or clause 9, wherein the at least one pressure sensor is positioned at the back area, and the pressure exerted on the patient is a pressure exerted on the patient's back.
11. The patient support apparatus of any one of clauses 8 to 10, wherein the at least one pressure sensor is positioned at the leg area, and the pressure exerted on the patient is a pressure exerted on the patient's legs.
12. The patient support apparatus of any one of clauses 8 to 11, wherein the at least one pressure sensor is positioned at the foot area, and the pressure exerted on the patient is a pressure exerted on the patient's feet.
13. The patient support apparatus of any one of clauses 8 to 12, wherein the at least one pressure sensor includes:
   a first pressure sensor positioned in at least one of the back area, the leg area, and the foot area, and
   a second pressure sensor positioned in another of the back area, the leg area, or the foot area.
14. The patient support apparatus of any one of clauses 8 to 13, wherein the at least one pressure sensor includes:
   a first pressure sensor positioned in the back area,
   a second pressure sensor positioned in the leg area, and
   a third pressure sensor positioned in the foot area.
15. The patient support apparatus of any preceding clause, wherein the risk areas are areas of the patient that are prone to pressure sores.
16. A patient support apparatus comprising:
   a mattress formed from a support material,
   a plurality of slots formed in the support material,
   a plurality of air bladders, each air bladder positioned within a slot,
   a cavity formed in the support material, and
   a pump configured to be positioned within the cavity, the pump being fluidly coupled to the plurality of air bladders when the pump is positioned within the cavity, the pump being configured to inflate and deflate the plurality of air bladders.
17. The patient support apparatus of clause 16 including at least one manifold having an inlet to receive air from the pump, the manifold having a plurality of outlets to discharge the air into the plurality of air bladders.
18. The patient support apparatus of clause 17, wherein the at least one manifold includes:
   a first manifold fluidly coupled to a first plurality of air bladders,
   a second manifold fluidly coupled to a second plurality of air bladders.
19. The patient support apparatus of clause 18, wherein the first manifold and the second manifold are each fluidly coupled to the pump.
20. The patient support apparatus of either clause 18 or clause 19, wherein each air bladder of the first plurality of air bladders is positioned between adjacent air bladders of the second plurality of air bladders.
21. The patient support apparatus of either clause 18 or clause 19, wherein the first plurality of air bladders is positioned in a first section of the mattress, and the second plurality of air bladders is positioned in a second section of the mattress.
22. The patient support apparatus of any one of clauses 16 to 21, wherein the support material includes foam.
23. The patient support apparatus of any one of clauses 16 to 22, wherein the plurality of slots are formed in the support material so that each slot of the plurality of slots is separated by a section of support material.
24. The patient support apparatus of any one of clauses 16 to 23, wherein each slot of the plurality of slots extends between a left side and a right side of the mattress.
25. The patient support apparatus of any one of clauses 16 to 24, wherein each slot extends from an opening formed in a first side of the mattress to a manifold positioned in a second side of the mattress.
26. The patient support apparatus of clause 25, wherein each air bladder of the plurality of air bladders is removably positioned within a respective slot.
27. The patient support apparatus of clause 26, wherein each air bladder of the plurality of air bladders is configured to be inserted into the opening formed in the first side of the mattress and fluidly coupled to the manifold positioned in the second side of the mattress.
28. The patient support apparatus of any one of clauses 16 to 27, wherein the pump is removably inserted into the cavity.
29. The patient support apparatus of clause 28, wherein the cavity extends from a cavity opening formed in a first side of the mattress to a manifold positioned in a second side of the mattress.
30. The patient support apparatus of clause 29, wherein the pump fluidly couples to the manifold when the pump is positioned within the cavity.
31. A patient support apparatus comprising:
   a mattress having a first side and a second side,
   a first turn-assist air bladder positioned on the first side,
   a second turn-assist air bladder positioned on the second side,
   a first inlet extending through the first side and in fluid communication with the first turn-assist air bladder,
   a second inlet extending through the second side and in fluid communication with the second turn-assist air bladder, and
   a pump that is configured to selectively couple to one of the first inlet and the second inlet.
32. The patient support apparatus of clause 31, wherein the pump is a hands-free pump and is operable to pump air without the use of hands.
33. The patient support apparatus of clause 32, wherein the pump is foot activated and configured to position on the floor.
34. The patient support apparatus of clause 33, further comprising a hose extending from the pump, the hose being configured to be coupled to one of the first inlet and the second inlet.
35. The patient support apparatus of any one of clauses 31 to 34, wherein the pump is configured to couple to the first inlet to inflate the first turn-assist air bladder.
36. The patient support apparatus of any one of clauses 31 to 35, wherein the pump is configured to couple to the second inlet to inflate the second turn-assist air bladder.
37. The patient support apparatus of any one of clauses 31 to 36, wherein the first turn-assist air bladder and the second turn-assist air bladder are positioned within the mattress.
38. The patient support apparatus of any one of clauses 31 to 37, wherein the first turn-assist air bladder and the second turn-assist air bladder are positioned under the mattress.
39. The patient support apparatus of any one of clauses 31 to 38, further comprising a timer that tracks a period of time since a patient was turned.
40. The patient support apparatus of clause 39, wherein the timer is positioned within the mattress and includes a display.
41. The patient support apparatus of either clause 39 or clause 40, wherein the timer is configured to be set so that a reminder is issued to turn the patient after a predetermined time period.
42. The patient support apparatus of clause 41, further comprising an input to select the predetermined time period.
43. The patient support apparatus of either clause 41 or clause 42, wherein the timer displays a first color during the predetermined time period.
44. The patient support apparatus of clause 43, wherein the timer displays a second color after the expiration of the predetermined time period.
45. The patient support apparatus of any one of clauses 39 to 44, wherein the timer resets after the patient is turned.
46. A patient support apparatus comprising:
   a mattress positioned on a frame,
   a plurality of air bladders positioned in the mattress and configured to be inflated and deflated to adjust a position of a patient on the mattress,
   a plurality of sensors configured to measure data related to the patient,
   a control system that is configured to track movement of the patient based on operation of the plurality of bladders and the data related to the patient, and
   a display configured to display the movement tracked by the control system in a graph, wherein a caregiver can review the graph to determine treatment for the patient.
47. The patient support apparatus of clause 46, wherein at least one of the plurality of air bladders is a therapeutic air bladder configured to provide pulsations or vibrations to the patient, wherein the control system tracks therapy provided for the patient and the graph includes an indicator representing the therapy.
48. The patient support apparatus of either clause 46 or clause 47, wherein at least one of the plurality of air bladders is a turn-assist air bladder configured to aid turning the patient, wherein the control system tracks patient turn assists and the graph includes an indicator representing patient turn assists.
49. The patient support apparatus of clause 48, wherein the graph includes a time stamp that identifies the time of a last patient turn assist.
50. The patient support apparatus of either clause 48 or clause 49, further comprising an input that enables a caregiver to identify a type of patient care that resulted in the patient turn assist.
51. The patient support apparatus of clause 50, wherein the type of patient care includes at least one of turning the patient, repositioning the patient, patient out of bed, skin assessment, hygiene care, and wound care.
52. The patient support apparatus of either clause 50 or clause 51, wherein the graph includes a label identifying the type of patient care.
53. The patient support apparatus of any one of clauses 46 to 52, wherein the plurality of sensors includes a pressure sensor, wherein the pressure sensor detects patient movement on the mattress based on a pressure detected by the pressure sensor.
54. The patient support apparatus of clause 53, wherein the pressure sensor detects when the patient is exiting the mattress.
55. The patient support apparatus of clause 54, wherein at least one of the plurality of air bladders is inflated to assist the patient in exiting the mattress.
56. The patient support apparatus of any one of clauses 46 to 55, wherein the display indicates a patient mobility level.
57. The patient support apparatus of clause 56, wherein the patient mobility level includes at least one of tonic, weak, and inert.
58. The patient support apparatus of either clause 56 or clause 57, wherein the patient mobility level includes a color coded graph.
59. The patient support apparatus of any one of clauses 46 to 58, wherein the display is coupled to the frame.
60. The patient support apparatus of any one of clauses 46 to 59, wherein the display includes a remote display.
61. A patient support apparatus comprising:
   a frame,
   a control system coupled to the frame and having a database including troubleshooting data,
   an input that enables a user to select troubleshooting data, and
   a display configured to display the troubleshooting data.
62. The patient support apparatus of clause 61, wherein the troubleshooting data includes data related to maintenance of the patient support apparatus.
63. The patient support apparatus of clause 62, wherein the troubleshooting data includes an augmented reality display.
64. The patient support apparatus of either clause 62 or clause 63, wherein the troubleshooting data includes instructional videos.
65. The patient support apparatus of any one of clauses 61 to 64, wherein the input is positioned on the display.
66. The patient support apparatus of any one of clauses 61 to 65, wherein the display is coupled to the frame.
67. The patient support apparatus of any one of clauses 61 to 66, wherein the display is positioned on a mobile device.

## Claims

1. A patient support apparatus comprising:
a seat section,
a head section rotatably coupled to the seat section, and
a leg section rotatably coupled to the seat section, wherein the seat section, head section, and leg section combine to form a support surface for a patient, the support surface having a back area where the patient's back is positioned when the patient is positioned on the support surface, a leg area where the patient's legs are positioned when the patient is positioned on the support surface, and a foot area where the patient's legs are positioned when the patient is positioned on the support surface,
at least one sensor positioned in at least one of the back area, leg area, and foot area, the at least sensor determining at least one angle of a patient position on the support surface, and
a control system coupled to the at least one sensor to determine risk areas for the patient based on the at least one angle of the patient position.

2. The patient support apparatus of claim 1, wherein the at least one sensor is positioned at the back area, and the at least one angle of the patient position is an angle of the patient's back relative to a floor.

3. The patient support apparatus of either claim 1 or claim 2, wherein the at least one sensor is positioned at the leg area, and the at least one angle of the patient position is an angle of the patient's legs relative to a floor.

4. The patient support apparatus of any preceding claim, wherein the at least one sensor is positioned at the foot area, and the at least one angle of the patient position is an angle of the patient's feet relative to a floor.

5. The patient support apparatus of any preceding claim, wherein the at least one sensor includes:
a first sensor positioned in at least one of the back area, the leg area, and the foot area, and
a second sensor positioned in another of the back area, the leg area, or the foot area.

6. The patient support apparatus of any preceding claim, wherein the at least one sensor includes:
a first sensor positioned in the back area,
a second sensor positioned in the leg area, and
a third sensor positioned in the foot area.

7. The patient support apparatus of any preceding claim, wherein the at least one sensor includes an accelerometer.

8. The patient support apparatus of any preceding claim, further comprising at least one pressure sensor positioned in at least one of the back area, the leg area, and the foot area, the at least one pressure sensor determining a pressure exerted on the patient.

9. The patient support apparatus of claim 8, wherein the control system determines risk areas for the patient based on a determined pressure.

10. The patient support apparatus of either claim 8 or claim 9, wherein the at least one pressure sensor is positioned at the back area, and the pressure exerted on the patient is a pressure exerted on the patient's back.

11. The patient support apparatus of any one of claims 8 to 10, wherein the at least one pressure sensor is positioned at the leg area, and the pressure exerted on the patient is a pressure exerted on the patient's legs.

12. The patient support apparatus of any one of claims 8 to 11, wherein the at least one pressure sensor is positioned at the foot area, and the pressure exerted on the patient is a pressure exerted on the patient's feet.

13. The patient support apparatus of any one of claims 8 to 12, wherein the at least one pressure sensor includes:
a first pressure sensor positioned in at least one of the back area, the leg area, and the foot area, and
a second pressure sensor positioned in another of the back area, the leg area, or the foot area.

14. The patient support apparatus of any one of claims 8 to 13, wherein the at least one pressure sensor includes:
a first pressure sensor positioned in the back area,
a second pressure sensor positioned in the leg area, and
a third pressure sensor positioned in the foot area.

15. The patient support apparatus of any preceding claim, wherein the risk areas are areas of the patient that are prone to pressure sores.
